# EUROPEAN PATENT SPECIFICATION

(11) **EP 4 262 525 B1**
(45) Date of publication and mention of the grant of the patent: **28.05.2025**
(21) Application number: 21830438.4
(22) Date of filing: 17.12.2021
(51) Int. Cl.: A61B 5/00, A61B 5/107

(54) **METHOD FOR MODELLING A NASAL CAVITY**
VERFAHREN ZUR MODELLIERUNG EINER NASENHÖHLE
PROCÉDÉ DE MODÉLISATION D'UNE CAVITÉ NASALE

(30) Priority: 18.12.2020 EP 20215527
(43) Date of publication of application: 25.10.2023
(73) Proprietor: Universiteit Antwerpen, 2000 Antwerpen (BE)
(72) Inventor: KEUSTERMANS, William, 2500 Lier (BE)
(74) Representative: IP HILLS NV
(86) International application number: PCT/EP2021/086378
(87) International publication number: WO 2022/129458

(56) References cited:
- US-A1- 2015 265 752
- KEUSTERMANS WILLIAM ET AL: "High quality statistical shape modelling of the human nasal cavity and applications", vol. 5, no. 12, 1 December 2018 (2018-12-01), pages 181558, XP055799280, Retrieved from the Internet <URL:https://royalsocietypublishing.org/doi/pdf/10.1098/rsos.181558> [retrieved on 20210628], DOI: 10.1098/rsos.181558
- KEUSTERMANS WILLIAM ET AL: "Matlab toolbox for semi-automatic segmentation of the human nasal cavity based on active shape modeling", COMPUTERS IN BIOLOGY AND MEDICINE, vol. 105, 1 February 2019 (2019-02-01), pages 27 - 38, XP085588924, ISSN: 0010-4825, DOI: 10.1016/J.COMPBIOMED.2018.12.008
- KUO CHUNG-FENG JEFFREY ET AL: "Application of intelligent automatic segmentation and 3D reconstruction of inferior turbinate and maxillary sinus from computed tomography and analyze the relationship between volume and nasal lesion", BIOMEDICAL SIGNAL PROCESSING AND CONTROL, ELSEVIER, AMSTERDAM, NL, vol. 57, 6 December 2019 (2019-12-06), XP086012281, ISSN: 1746-8094, [retrieved on 20191206], DOI: 10.1016/J.BSPC.2019.101660

## Description

### Field of the Invention

The present invention generally relates to a computer-implemented method for modelling a nasal cavity.

### Background of the Invention

Nasal airway obstruction (NAO) is a relatively frequent problem encountered by ear-nose-throat (ENT or ORL) physicians. Increasing incidence of allergy additionally increases the number of patients with nasal obstruction. Septoplasty and turbinate surgery appear to be treatments of choice in case of anatomic abnormalities and are among the most frequently performed operations by ENT surgeons. The decision to proceed to surgery is generally based on a surgeon's assessment. This assessment is impeded by a poor correlation between existing objective tests on the one hand, such as tomographic imagery, visual inspection, rhinomanometry and/ or acoustic rhinometry, and patient's symptoms or patient's subjective feelings on the other hand. Given this impediment and the complex nasal anatomy, it is not surprising that the overall results for long-term relief of NAO and associated improvement of quality of life are very unsatisfactory.

An objective tool for a pre-operative assessment of the nasal cavity may be found in computational fluid dynamics (CFD). CFD is a technology which can allow researchers to predict airflow throughout the nasal cavity based on a three-dimensional model of the nasal cavity which may be derived from for example tomographic imaging (e.g. CT-scans). However, the conversion from tomographic data to a model of the nasal cavity has proven to be a labour-intensive task, inhibiting its clinical application.

As an example, Keustermans et al (2018-12-01) disclose a technique to provide a more comprehensive nasal cavity shape model which is derived from individual models generated from CT data of a limited number of patients. However, the technique disclosed in the paper cannot be put into practice for a large number of patients for the reason mentioned above. Moreover, the technique is still based on medical imagery.

An additional problem is formed by the fact that a nasal cavity does not have a static shape over time. The shape of the nasal cavity is subject to the so-called nasal cycle, which is a natural process in which the nasal mucosa periodically congests and decongests over a period of a couple of hours. The left and right nasal channel do this in antiphase, i.e. when the left nasal channel is congested, the right nasal channel is decongested and vice versa. A single tomographic imaging is therefore only a snapshot in time of the nasal anatomy. At the same time, multiple tomographic imaging is not feasible due to the danger of the radiation involved and/or due to the high costs of for example MRI scans.

Additionally, in the pharmaceutical industry, there is a growing interest in nasal drug delivery. However, current nasal drug delivery devices appear to perform sub-optimally. In this application as well, CFD simulations may potentially improve performance of nasal drug delivery devices. But obtaining tomographic imagery of the nasal cavity may not be feasible in this field since there may not be a medical indication to perform such imagery.

### Summary of the Invention

It is therefore an aim of the present invention to solve or at least alleviate one or more of the above-mentioned problems. In particular, the invention aims at providing an improved model for describing a specific nasal cavity shape as well as a method for modelling a specific nasal cavity shape in a relatively fast and cost-efficient manner allowing subsequent CFD being performed thereon and possibly avoiding any radiation due to imaging.

Disclosed is a non-claimed computer-implemented model for describing a specific nasal cavity shape. The model comprises a generic nasal cavity shape model including an average nasal cavity shape and a set of nasal cavity shape eigenmodes. The model further comprises a set of specific parameters such that the specific nasal cavity shape is modelled by a combination, in particular by a sum, of the average nasal cavity shape and a linear combination of the set of specific parameters with the set of nasal cavity shape eigenmodes. Said set of specific parameters is derived from measurement data of the specific nasal cavity, in particular from measurement data on quasi static and/or dynamic nasal pressure changes. Such measurement data may for example encompass data on quasi static nasal pressure changes, such as rhinomanometry data, and/or dynamic nasal pressure changes such as acoustic rhinometry data. In the context of the present application, the term 'specific' refers to what is linked to a specific person. As explained above, nasal cavity shapes can vary widely between different persons, as well as over time for a specific person. A general nasal cavity model may therefor differ substantially from a specific nasal cavity of a specific person at a given moment in time. Since the present model can describe a specific nasal cavity shape based only on a generic nasal cavity model in combination with measurement data of the specific nasal cavity of a given person, the model can provide a solution to the above-identified problem, without the need for additional tomographic imaging, which may be a costly and time-consuming procedure.

The average nasal cavity shape and the set of nasal cavity shape eigenmodes may be 3D surface representations of nasal cavities. These 3D surface representations are preferably point clouds in which the points are distributed over a surface or boundary of the nasal cavity shape. These surface representations can allow further identifications of corresponding points located on the same anatomical position between surface representations of different nasal cavity shapes. Alternatively, these 3D surface representations may also be surface meshes including edges and faces configured to indicate a degree of connectivity between points.

Disclosed is a non-claimed computer-implemented method for modelling a specific nasal cavity shape. The method comprises the steps of obtaining measurement data of a nasal cavity and feeding said measurement data into a neural network. Said measurement data include data on quasi static and/or dynamic nasal pressure changes, such as for example rhinomanometry data or acoustic rhinometry data. The neural network is trained to output a set of specific parameters such that the specific nasal cavity shape is modelled by a combination, in particular by a sum, of an average nasal cavity shape and a linear combination of the set of specific parameters with a set of nasal cavity shape eigenmodes. As mentioned above, the present method can provide a specific nasal cavity model based only measurement data which are relatively easy to obtain from a person, such as acoustic rhinometry or rhinomanometry data, and an inventive combination of such data with a generic nasal cavity model.

The obtaining of measurement data can preferably include obtaining acoustic rhinometry measurement data. Such measurement data can provide a cross-sectional surface area in function of a depth of at least one of a right nasal channel and a left nasal channel of a nasal cavity of a person. This is a well-known measurement technique in ORL practice, for example in allergen provocation tests. The size and pattern of reflected sound waves can provide information on the structure and dimensions of the nasal cavity, with the time delay of reflections correlating with the distance from the nostril. Such measurements have been shown to correlate relatively well with measurements on CT scans. Moreover, these acoustic rhinometry measurement data can be obtained in a non-invasive way. Alternatively, rhinomanometry measurements could be used.

According to an aspect of the invention, there is provided a computer-implemented method of training a neural network to output a set of specific parameters derived from measurement data of a nasal cavity such that a specific nasal cavity shape is modelled by a combination, in particular by a sum, of an average nasal cavity shape and a linear combination of the set of specific parameters with a set of nasal cavity shape eigenmodes. The training of the neural network includes the steps of randomly generating sets of specific parameters simulating measurement data of nasal cavities. Then specific nasal cavity shape models are generated, said models including a combination of an average nasal cavity shape and a linear combination of said simulated sets of specific parameters with a set of nasal cavity shape eigenmodes. Next, a cross-sectional surface area of at least one of the nasal channels of the specific nasal cavity shape models provided by said simulated sets of specific parameters is determined. The determined cross-sectional surface area of at least one of the nasal channels is then fed into the neural network. Finally, the neural network is trained to output the sets of specific parameters, which had been randomly generated. In this way, the neural network can be trained relatively easily by simulated data which are based on randomly generated sets of specific parameters. No additional measurements or imagery on a person is needed. Alternatively, the training can be done by labelled data based on real measurement data, for example acoustic rhinometry data, from a person for whom data obtained from for example tomographic imaging is present.

The generating specific nasal cavity shape models can include obtaining a generic nasal cavity shape model. Such a generic nasal cavity shape model can be obtained by generating 3D surface representations of a plurality of nasal cavities. Said 3D surface representations may for example be clouds of points. Each 3D surface representation includes a same number of points. Next, corresponding points between said 3D surface representations of the plurality of nasal cavities are being identified such that said corresponding points are located on a same anatomic position. Then an average nasal cavity shape is generated based on average values of said corresponding points. From said 3D surface representations a set of nasal cavity shape eigenmodes is extracted. The generic nasal cavity shape model can then include the average nasal cavity shape and the set of nasal cavity shape eigenmodes. In this way, even if the generation of a generic nasal cavity shape model may have been relatively labour- or cost-intensive, the generic nasal cavity shape model can now allow a relatively easy generation of specific nasal cavity shape models with relatively little effort.

The generating of 3D surface representations may for example be based on tomographic images of a plurality of nasal cavities. The tomographic images may be images of pathological or non-pathological nasal cavities. Databases of tomographic images in ORL centres or hospitals may be used to obtain a relatively large amount of tomographic images of various nasal cavities. Any other kind of available data, for example other imaging data, may also be used to generate said 3D surface representations.

The generating of 3D surface representations can include mirroring said 3D surface representations. Since nasal cavities are asymmetric, and the left nasal cavity channel is not a mirror image of the right nasal cavity channel, each mirror image can represent an additional 3D surface representation. In this way, the number of available 3D surface representations can be easily doubled without the need for more imagery on people.

The finding of corresponding points can for example include applying a cylindrical parametrization technique for mapping tubular surfaces. It has been found that the shape of one of the nasal channels followed by the other of the nasal channel can be approximated relatively well by a tubular surface, which can simplify parametrization of the surface. An example of such a cylindrical parametrization technique has been disclosed in WO 2010/142624, which has the advantage of being able to map a relatively complex topology, such as of a nasal cavity shape, onto a cylinder on which calculations can be speeded up. Said technique can allow to decrease stretch distortions. Other parametrization techniques may also be used.

The generating of the average nasal cavity shape and the extracting the set of nasal cavity shape eigenmodes is done by applying a principal component analysis. This is a well-known dimensionality reduction technique which can be implemented in a relatively efficient way. The set of nasal cavity shape eigenmodes is preferably an orthogonal set of eigenmodes to reduce computation time and to ensure that each nasal cavity shape model can be attributed a unique set of specific parameters, which can favour an accurate training of the neural network. Other dimensionality reduction techniques may be used instead, such as for example an independent component analysis.

Disclosed is a non-claimed computer-implemented neural network for modelling a specific nasal cavity shape. The neural network comprises a generic nasal cavity shape model including an average nasal cavity shape and a set of nasal cavity shape eigenmodes. The neural network is trained to output a set of specific parameters derived from measurement data, in particular measurement data on quasi static and/or dynamic nasal pressure changes, of the specific nasal cavity such that the specific nasal cavity shape is modelled by a combination of the average nasal cavity shape and a linear combination of the set of specific parameters with the set of nasal cavity shape eigenmodes. Such a neural network can provide an efficient tool for ORL specialists: only by feeding person-specific measurement data into the neural network, the neural network can provide a specific nasal cavity shape model in a relatively fast manner, which model can then help an ORL practitioner to decide on a treatment if needed. In industry and research, for example in pharmaceutical industry, such specific nasal cavity shape models can help in deciding on person-specific dosages of medical nasal sprays or on administration strategies. Since the specific nasal cavity shape model is a computer-implemented model, the model can further be used to perform computational fluid dynamics calculations on the model. The neural network can for example be trained as previously described, providing one or more of the described advantages.

### Brief Description of the Drawings

Fig. 1a illustrates the step of obtaining measurement data of a nasal cavity of the method for modelling a specific nasal cavity shape according to an aspect of the invention;
Fig. 1b shows a schematic graph showing the measurement data of the step illustrated in Fig. 1a;
Fig. 2 shows a schematic cross-sectional view of a nasal cavity to be modelled;
Fig. 3 shows a schematic representation of a neural network;
Fig. 4 shows a schematic representation of the model for describing a specific nasal cavity shape;
Fig. 5 shows a perspective view and a top view on a 3D representation of a nasal cavity;
Fig. 6a - 6e show a preferred embodiment of the step of finding corresponding points between 3D surface representations of the method of training a neural network according to an aspect of the invention;
Fig. 7 shows a preferred embodiment of the step of extracting from 3D surface representations a set of nasal cavity shape eigenmodes;
Fig. 8 shows a preferred embodiment of the method of training a neural network according to an aspect of the invention; and
Fig. 9 shows a computing system suitable for performing various steps of the method for modelling a specific nasal cavity shape according to an aspect of the invention.

### Detailed Description of Embodiment(s)

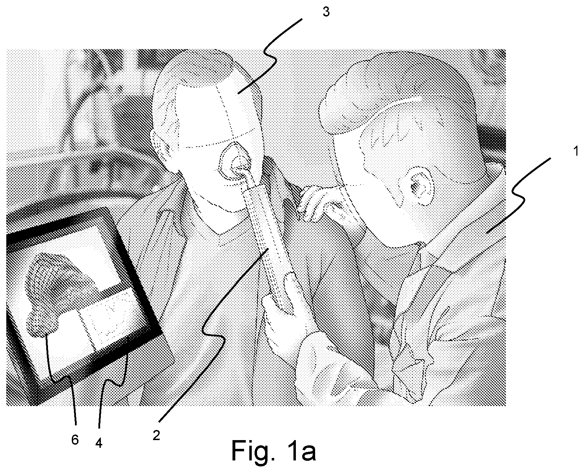

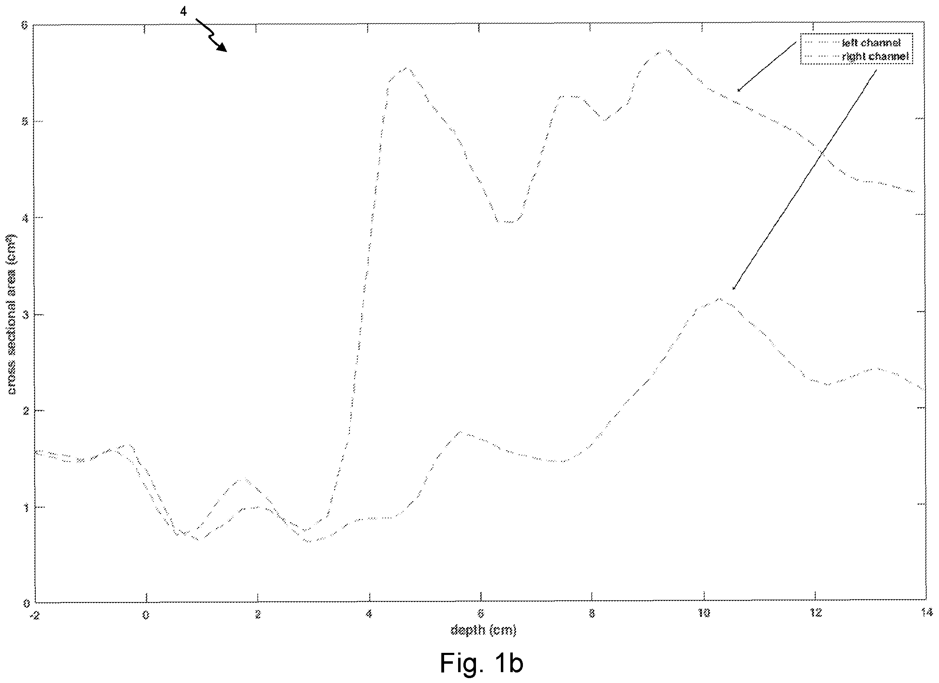

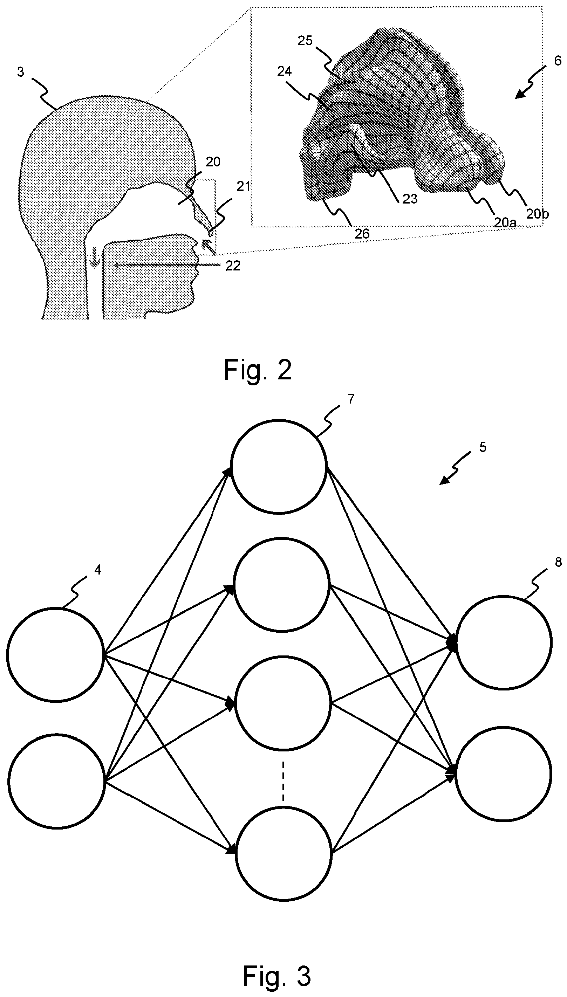

Figure 1a illustrates the step of obtaining measurement data of a nasal cavity of the method for modelling a specific nasal cavity shape. Measurement data can for example be obtained by acoustic rhinometry. A practitioner 1 uses a dedicated device 2 configured to generate and emit acoustic waves into a nasal channel of a person 3. The acoustic waves are reflected within the nasal channel. The device 2 is further configured to detect a reflection by the nasal channel of said emitted acoustic waves. Time delays between emission and detection of reflected acoustic waves can then provide information on a cross-sectional area in function of depth per nasal channel as illustrated in Figure 1b showing a schematic graph of the measurement data 4. These measurement data 4 are then fed into a neural network 5, as will be illustrated in Figure 3. The neural network 5 is trained to output a set of specific parameters such that the specific nasal cavity shape of the person 3 is modelled by a combination of an average nasal cavity shape and a linear combination of the set of specific parameters with a set of nasal cavity shape eigenmodes. The model for describing the specific nasal cavity shape 6 can then be visualized by the practitioner 1, and/or the practitioner 1 can do calculations, for example CFD calculations on the specific model 6.

Fig. 2 shows a schematic cross-sectional view of a nasal cavity 20 to be modelled, as well as a perspective view on a model 6 of said nasal cavity 20. The nasal cavity is a large air-filled space behind the nose 21 and forms the upper part of the respiratory system. The nasal cavity is divided into two nasal channels or fossae 20a, 20b through which air can flow between the nostrils and the pharynx 22 and the rest of the respiratory tract. The nasal cavity 20 can further include an inferior meatus or passage 23, a middle meatus 24, a superior meatus 25, and in some cases a supreme meatus. A model of the nasal cavity may also comprise the nasopharynx 26, connecting the nasal cavity with the pharynx. The model may comprise the entire nasal cavity or only part of it, with or without the nasopharynx, or for example including only one nasal channel or part of it. Nasal functions can include olfaction, ventilation, heating and/or humidification of inhaled air and filtration of potentially harmful particles. These nasal functions can be severely impaired in case of nasal airway obstruction, which may for example be caused by anatomic deformities such as a septal deviation or perforation, an enlarged turbinate or others.

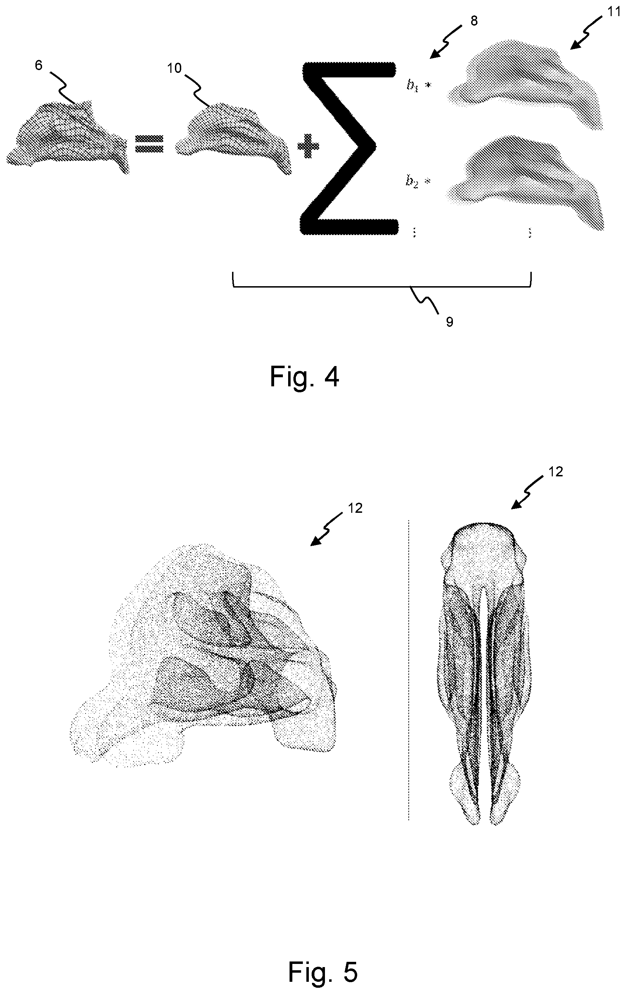

Fig. 3 shows a schematic representation of a neural network. As explained before, measurement data 4, such as for example acoustic measurement data obtained by a practitioner 1 of a nasal cavity of a person 3, are input into the neural network 5. The neural network 5 has been trained, or in other words, parameters of at least one hidden layer 7 have been tweaked, such that the neural network 5 outputs a set of specific parameters 8. The specific nasal cavity shape of the person 3 can then be modelled by a combination of an average nasal cavity shape and a linear combination of said set of specific parameters 8 output by the trained neural network 5 with a set of nasal cavity shape eigenmodes, as will be explained under Figure 4.

Fig. 4 shows a schematic representation of the model for describing a specific nasal cavity shape. The model 6 comprises a generic nasal cavity shape model 9 including an average nasal cavity shape 10 and a set of nasal cavity shape eigenmodes 11. The model 6 further comprises a set of specific parameters 8 such that the nasal cavity shape of a specific person 3 is modelled by a combination, in particular by a sum, of the average nasal cavity shape 10 and a linear combination of the set of specific parameters 8 with the set of nasal cavity shape eigenmodes 11. In an inventive way, the set of specific parameters 8 is derived from measurement data 4 of the nasal cavity 20 of a specific person 3, preferably by a neural network 5 trained thereto. The average nasal cavity shape 10 and the set of nasal cavity shape eigenmodes 11 are preferably based 3D surface representations 12 of nasal cavities, as will be shown in Figure 5.

Fig. 5 shows a perspective view and a top view respectively on a 3D representation 12 of a nasal cavity. Such a 3D representation can for example be based on tomographic images of a nasal cavity. In order to build a generic nasal cavity shape model 9, a relatively large number, for example between 50 to 100 or preferably many more, of tomographic images, such as for example CT scans, may be used to generate 3D surface representations of said plurality of nasal cavities. Each 3D representation can preferably include a same number of points, for example 100 000 points or more or less, which are distributed over the nasal cavity's boundaries or surface. Since nasal cavities are not symmetric, the 3D surface representations may be mirrored to increase a variety in the sample of nasal cavities. Starting from said 3D surface representations, corresponding points between representations of different nasal cavities can be identified which are located on the same anatomical positions on all 3D surface representations.

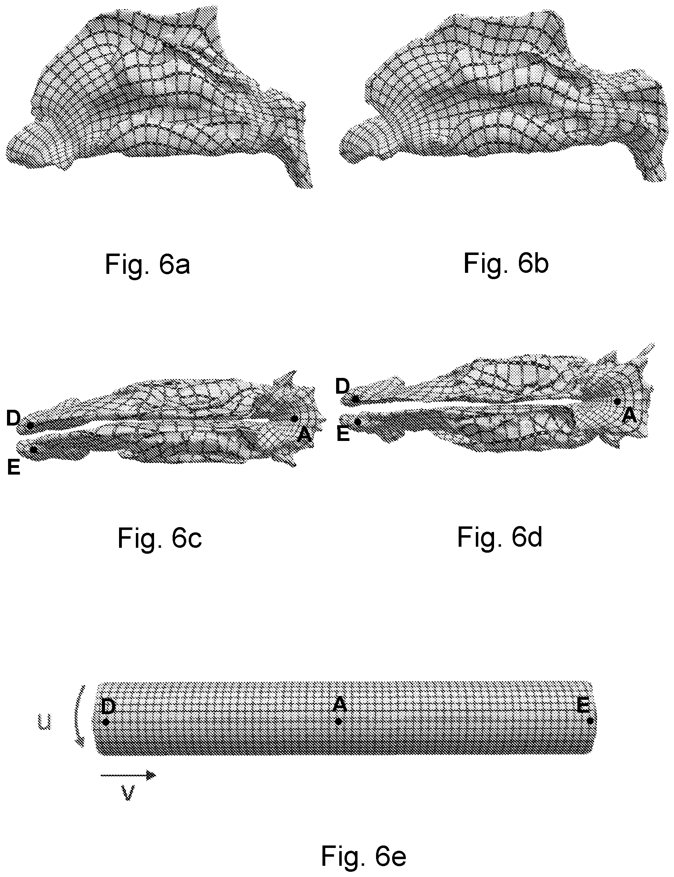

Fig. 6a - 6e show a preferred embodiment of the step of finding corresponding points between 3D surface representations of the method of training a neural network, in particular of the method of obtaining a generic nasal cavity shape model, according to an aspect of the invention. Figures 6a and 6b show a sagittal view on a nasal cavity model of two different subjects, whereas Figures 6c and 6d show an axial or top view on the respective nasal cavity models of Figures 6a and 6b. The models have been derived from 3D surface representations as shown in Figure 5. A surface parametrization has then been applied to all 3D surface representations. Each nasal cavity has been mapped onto a cylindrical surface such as shown in Figure 6e, as if a nasal cavity were a long cylinder folded as a U-shape from a first nasal channel over the nasopharynx to the other nasal channel, as can be seen in the top views of Figures 6c and 6d. Locations of corresponding points, for example of points A, D, E, and many others, can then be expressed in a cylindrical coordinate system (u, v). To make sure that all meshes include a same number of vertices located at the same anatomical position, a minimum description length correspondence optimization has been applied, such as for example described in WO 2010/142624, which can result in the statistical shape models as shown in Figures 6a - 6d. In a next step, a principal component analysis has been applied on the vertices of the 3D surface representations.

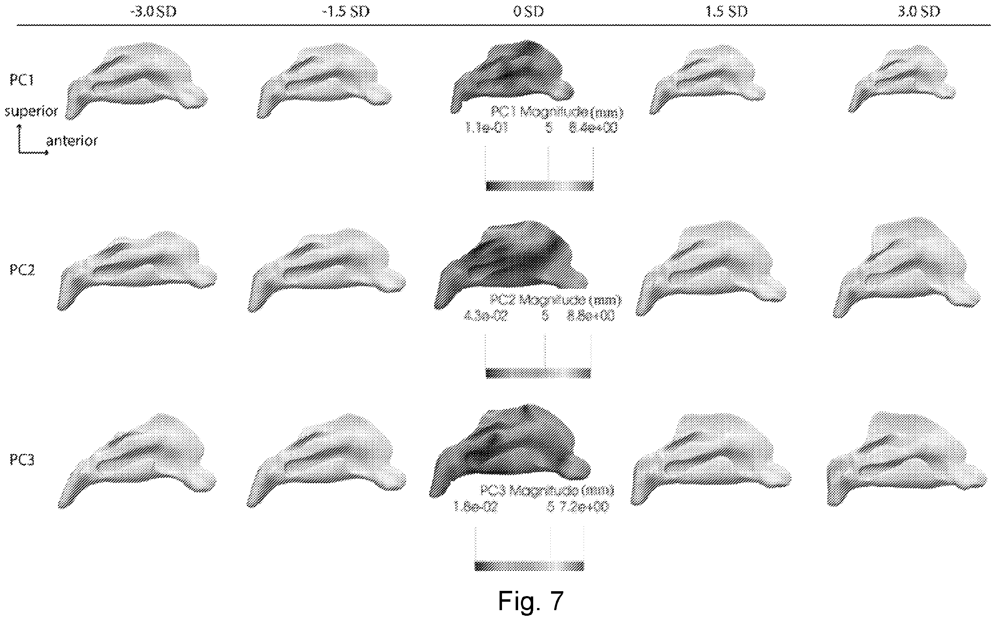

Fig. 7 shows a preferred embodiment of a set of nasal cavity shape eigenmodes resulting from the principal component analysis (PCA) on the vertices of the 3D surface representations 12 of the plurality of nasal cavities. The PCA has generated an average nasal cavity shape 10 based on average values of said corresponding points, such as for example A, D, E. The PCA has also extracted a set, preferably an orthogonal set, of nasal cavity shape eigenmodes 11 from said 3D surface representations. Said set of nasal cavity shape eigenmodes 11 can represent the shape variations or deformations with respect to the average nasal cavity shape 10. In Figure 7, an example of the first three shape eigenmodes is given. The colour map represents a magnitude of variation for the corresponding principal component. The specific shape parameters can be expressed as a multiple of a standard deviation of a distribution of the eigenvalues linked to a given eigenvector. Since higher principal components represent smaller shape variations, most variation can be represented with a limited number of parameters, for example based on the first 20 to 40 principal components, more preferably on the first 20 - 25 principal components.

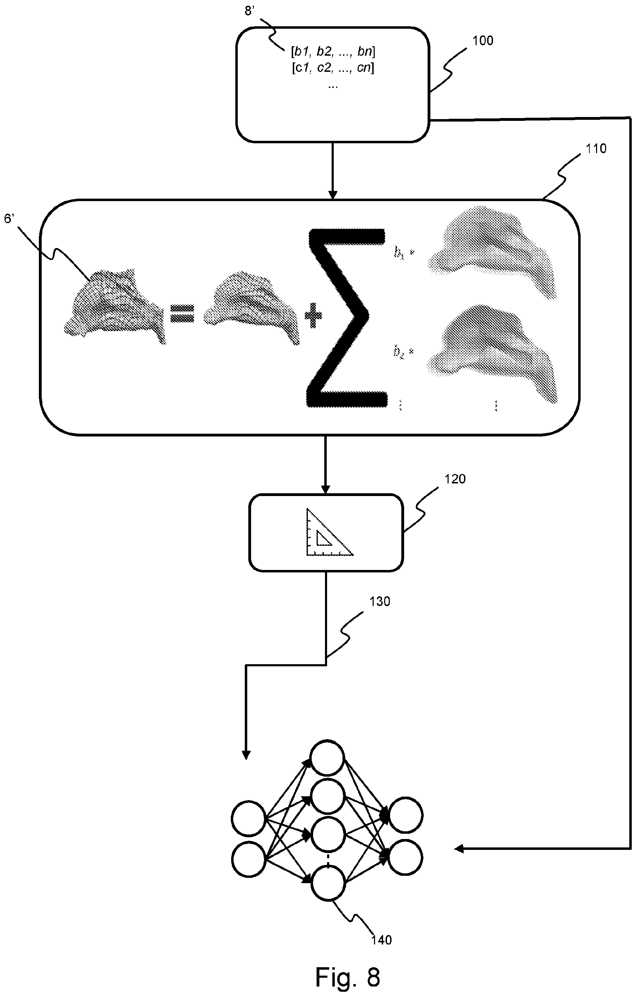

This generic nasal cavity shape model 9 now allows to model any specific nasal cavity shape as a combination, in particular as a sum, of an average nasal cavity shape 10 and a linear combination of the set of specific parameters 8 with a set of nasal cavity shape eigenmodes 11, as shown in Figure 4. According to an aspect of the invention, said set of specific parameters 8 is based on measurement data on quasi static and/or dynamic nasal pressure changes, in particular on acoustic rhinometry measurement data 4, which are fed into the neural network 5. In order to train the neural network 5 to output said set of specific parameters 8, an aspect of the invention provides a method of training the neural network 5, of which a preferred embodiment is shown in Figure 8. In a first step 100, sets of specific parameters 8' are randomly generated simulating measurement data of nasal cavities. Then in step 110, specific nasal cavity shape models are generated by introducing said simulated sets of specific parameters 8' into the generic model 9 as shown in Figure 4. The generated specific nasal cavity shape models 6' thus include a combination of an average nasal cavity shape 10 and a linear combination of said simulated sets of specific parameters 8' with a set of nasal cavity shape eigenmodes 11. In a next step 120, a cross-sectional surface area of at least one of the nasal channels of the specific nasal cavity shape models 6' provided by said simulated sets of specific parameters 8' is determined, which can be done on the computer-implemented models 6'. Then in step 130 the determined cross-sectional surface area of at least one of the nasal channels is fed into the neural network 5, as for example shown in Figure 3. Finally, the neural network 5 is trained, i.e. network parameters 140 are tweaked, to output the sets of specific parameters 8' which had been randomly generated.

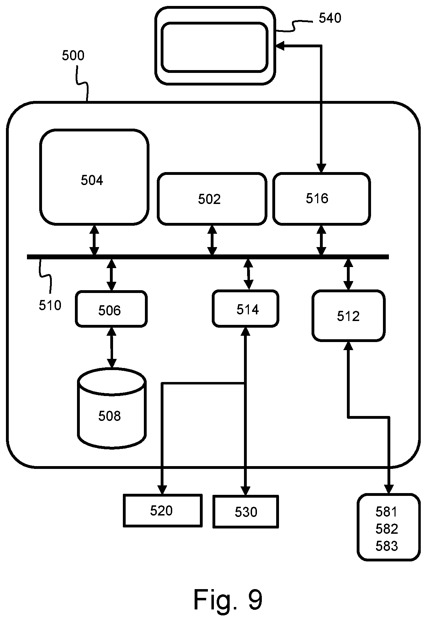

Figure 9 shows a suitable computing system 500 comprising circuitry enabling the performance of steps of embodiments of the method for modelling a specific nasal cavity shape according to an aspect of the invention, or the method for training a neural network according to another aspect of the invention. Computing system 500 may in general be formed as a suitable general-purpose computer and comprise a bus 510, a processor 502, a local memory 504, one or more optional input interfaces 514, one or more optional output interfaces 516, a communication interface 512, a storage element interface 506, and one or more storage elements 508. Bus 510 may comprise one or more conductors that permit communication among the components of the computing system 500. Processor 502 may include any type of conventional processor or microprocessor that interprets and executes programming instructions. Local memory 504 may include a random-access memory (RAM) or another type of dynamic storage device that stores information and instructions for execution by processor 502 and/or a read only memory (ROM) or another type of static storage device that stores static information and instructions for use by processor 502. Input interface 514 may comprise one or more conventional mechanisms that permit an operator or user to input information to the computing device 500, such as a keyboard 520, a mouse 530, a pen, voice recognition and/or biometric mechanisms, a camera, etc. Output interface 516 may comprise one or more conventional mechanisms that output information to the operator or user, such as a display 540, etc. Communication interface 512 may comprise any transceiver-like mechanism such as for example one or more Ethernet interfaces that enables computing system 500 to communicate with other devices and/or systems, for example with other computing devices 581, 582, 583. The communication interface 512 of computing system 500 may be connected to such another computing system by means of a local area network (LAN) or a wide area network (WAN) such as for example the internet. Storage element interface 506 may comprise a storage interface such as for example a Serial Advanced Technology Attachment (SATA) interface or a Small Computer System Interface (SCSI) for connecting bus 510 to one or more storage elements 508, such as one or more local disks, for example SATA disk drives, and control the reading and writing of data to and/or from these storage elements 508. Although the storage element(s) 508 above is/are described as a local disk, in general any other suitable computer-readable media such as a removable magnetic disk, optical storage media such as a CD or DVD, - ROM disk, solid state drives, flash memory cards, ... could be used.

As used in this application, the term "circuitry" may refer to one or more or all of the following:
(a) hardware-only circuit implementations such as implementations in only analog and/or digital circuitry and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions) and
(c) hardware circuit(s) and/or processor(s), such as microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in a server, a cellular network device, or other computing or network device.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A computer-implemented method of training a neural network (5) to output a set of specific parameters (8) derived from measurement data (4) of a nasal cavity such that a specific nasal cavity shape (6) is modelled by a combination of an average nasal cavity shape (10) and a linear combination of the set of specific parameters with a set of nasal cavity shape eigenmodes (11), the training including the steps of
- randomly generating (100) sets of specific parameters simulating measurement data of nasal cavities;
- generating (110) specific nasal cavity shape models, said models including a combination of an average nasal cavity shape and a linear combination of said simulated sets of specific parameters with a set of nasal cavity shape eigenmodes;
- determining (120) a cross-sectional surface area of at least one of the nasal channels of the specific nasal cavity shape models provided by said simulated sets of specific parameters;
- feeding (130) the determined cross-sectional surface area of at least one of the nasal channels into the neural network;
- training (140) the neural network to output the sets of specific parameters.

2. The method of claim 1, wherein the generating specific nasal cavity shape models includes obtaining a generic nasal cavity shape model (9), the obtaining comprising the steps of
- generating 3D surface representations of a plurality of nasal cavities, wherein each 3D surface representation includes a same number of points;
- finding corresponding points between said 3D surface representation, wherein said corresponding points are located on a same anatomic position;
- generating an average nasal cavity shape based on average values of said corresponding points;
- extract from said 3D surface representations a set of nasal cavity shape eigenmodes;
wherein the generic nasal cavity shape model (9) includes the average nasal cavity shape (10) and the set of nasal cavity shape eigenmodes (11).

3. The method according to claim 2, wherein the generating of 3D surface representations is based on tomographic images of the plurality of nasal cavities.

4. The method according to claim 2 or 3, wherein the generating of 3D surface representations includes mirroring said 3D surface representations.

5. The method according to any of the preceding claims 2-4, wherein the finding of corresponding points includes applying a cylindrical parametrization technique for mapping tubular surfaces.

6. The method according to any of the preceding claims 2-5, wherein the generating of the average nasal cavity shape and the extracting the set of nasal cavity shape eigenmodes is done by applying a principal component analysis.

7. A controller comprising at least one processor and at least one memory including computer program code, the at least one memory and computer program code configured to, with the at least one processor, cause the controller to perform the method according to any of the preceding claims 1-6.

8. A computer program product comprising computer-executable instructions for performing the method according to any of the preceding claims 1-6 when the program is run on a computer.

9. A computer readable storage medium comprising computer-executable instructions for performing the method according to any of the preceding claims 1-6 when the program is run on a computer.

## Patentansprüche

1. Computerimplementiertes Verfahren zum Trainieren eines neuronalen Netzes (5), um einen Satz spezifischer Parameter (8) auszugeben, die aus Messdaten (4) einer Nasenhöhle abgeleitet sind, sodass eine spezifische Nasenhöhlenform (6) durch eine Kombination einer durchschnittlichen Nasenhöhlenform (10) und einer linearen Kombination des Satzes spezifischer Parameter mit einem Satz von Nasenhöhlenform-Eigenmoden (11) modelliert wird, wobei das Training die folgenden Schritte enthält:
- zufälliges Erzeugen (100) von Sätzen spezifischer Parameter, die Messdaten von Nasenhöhlen simulieren;
- Erzeugen (110) spezifischer Nasenhöhlenformmodelle, wobei die Modelle eine Kombination einer durchschnittlichen Nasenhöhlenform und einer linearen Kombination der simulierten Sätze spezifischer Parameter mit einem Satz von Nasenhöhlenform-Eigenmoden enthalten;
- Bestimmen (120) einer Querschnittsoberfläche mindestens eines der Nasenkanäle der spezifischen Nasenhöhlenformmodelle, die durch die simulierten Sätze spezifischer Parameter bereitgestellt werden;
- Eingeben (130) der bestimmten Querschnittsoberfläche mindestens eines der Nasenkanäle in das neuronale Netz;
- Trainieren (140) des neuronalen Netzes, um die Sätze spezifischer Parameter auszugeben.

2. Verfahren nach Anspruch 1, wobei das Erzeugen spezifischer Nasenhöhlenformmodelle Erhalten eines generischen Nasenhöhlenformmodells (9) enthält, wobei das Erhalten die folgenden Schritte umfasst:
- Erzeugen von 3D-Oberflächendarstellungen einer Vielzahl von Nasenhöhlen, wobei jede 3D-Oberflächendarstellung eine gleiche Anzahl von Punkten enthält;
- Finden entsprechender Punkte zwischen der 3D-Oberflächendarstellung, wobei sich die entsprechenden Punkte an einer gleichen anatomischen Position befinden;
- Erzeugen einer durchschnittlichen Nasenhöhlenform basierend auf Durchschnittswerten der entsprechenden Punkte;
- Extrahieren eines Satzes von Nasenhöhlenform-Eigenmoden aus den 3D-Oberflächendarstellungen;
wobei das generische Nasenhöhlenformmodell (9) die durchschnittliche Nasenhöhlenform (10) und den Satz von Nasenhöhlenform-Eigenmoden (11) enthält.

3. Verfahren nach Anspruch 2, wobei das Erzeugen von 3D-Oberflächendarstellungen auf tomographischen Bildern der Vielzahl von Nasenhöhlen basiert.

4. Verfahren nach Anspruch 2 oder 3, wobei das Erzeugen von 3D-Oberflächendarstellungen Spiegeln der 3D-Oberflächendarstellungen enthält.

5. Verfahren nach einem der vorhergehenden Ansprüche 2-4, wobei das Finden entsprechender Punkte Anwenden einer zylindrischen Parametrisierungstechnik zum Abbilden röhrenförmiger Oberflächen enthält.

6. Verfahren nach einem der vorhergehenden Ansprüche 2-5, wobei das Erzeugen der durchschnittlichen Nasenhöhlenform und das Extrahieren des Satzes von Nasenhöhlenform-Eigenmoden durch Anwenden einer Hauptkomponentenanalyse erfolgt.

7. Steuerung, umfassend zumindest einen Prozessor und zumindest einen Speicher, der einen Computerprogrammcode enthält, wobei der zumindest eine Speicher und der Computerprogrammcode dazu konfiguriert sind, mit dem zumindest einen Prozessor zu bewirken, dass die Steuerung das Verfahren nach einem der vorhergehenden Ansprüche 1-6 durchführt.

8. Computerprogrammprodukt, umfassend computerausführbare Anweisungen zum Durchführen des Verfahrens nach einem der vorhergehenden Ansprüche 1-6, wenn das Programm auf einem Computer läuft.

9. Computerlesbares Speichermedium, umfassend computerausführbare Anweisungen zum Durchführen des Verfahrens nach einem der vorhergehenden Ansprüche 1-6, wenn das Programm auf einem Computer läuft.

## Revendications

1. Procédé mis en œuvre par ordinateur de formation d'un réseau neuronal (5) pour délivrer en sortie un ensemble de paramètres spécifiques (8) dérivés de données de mesure (4) d'une cavité nasale de sorte qu'une forme de cavité nasale spécifique (6) soit modélisée par une combinaison d'une forme de cavité nasale moyenne (10) et d'une combinaison linéaire de l'ensemble de paramètres spécifiques avec un ensemble de modes propres de forme de cavité nasale (11), la formation comprenant les étapes consistant à
- générer aléatoirement (100) des ensembles de paramètres spécifiques simulant des données de mesure de cavités nasales ;
- générer (110) des modèles de forme de cavité nasale spécifiques, lesdits modèles comprenant une combinaison d'une forme de cavité nasale moyenne et d'une combinaison linéaire desdits ensembles simulés de paramètres spécifiques avec un ensemble de modes propres de forme de cavité nasale ;
- déterminer (120) une aire de section transversale d'au moins un des canaux nasaux des modèles de forme de cavité nasale spécifiques fournis par lesdits ensembles simulés de paramètres spécifiques ;
- introduire (130) dans le réseau neuronal l'aire de section transversale déterminée d'au moins un des canaux nasaux ;
- former (140) le réseau neuronal pour délivrer en sortie les ensembles de paramètres spécifiques.

2. Procédé selon la revendication 1, ladite génération de modèles de forme de cavité nasale spécifiques comprenant l'obtention d'un modèle de forme de cavité nasale générique (9), ladite obtention comprenant les étapes consistant à
- générer des représentations de surface 3D d'une pluralité de cavités nasales, chaque représentation de surface 3D comprenant un même nombre de points ;
- rechercher des points correspondants entre ladite représentation de surface 3D, lesdits points correspondants étant situés sur une même position anatomique ;
- générer une forme de cavité nasale moyenne sur la base de valeurs moyennes desdits points correspondants ;
- extraire desdites représentations de surface 3D un ensemble de modes propres de forme de cavité nasale ;
ledit modèle de forme de cavité nasale générique (9) comprenant la forme de cavité nasale moyenne (10) et l'ensemble de modes propres de forme de cavité nasale (11).

3. Procédé selon la revendication 2, ladite génération de représentations de surface 3D étant basée sur des images tomographiques de la pluralité de cavités nasales.

4. Procédé selon la revendication 2 ou 3, ladite génération de représentations de surface 3D comprenant la mise en miroir desdites représentations de surface 3D.

5. Procédé selon l'une quelconque des revendications précédentes 2 à 4, ladite recherche de points correspondants comprenant l'application d'une technique de paramétrisation cylindrique pour cartographier des surfaces tubulaires.

6. Procédé selon l'une quelconque des revendications précédentes 2 à 5, ladite génération de la forme de cavité nasale moyenne et ladite extraction de l'ensemble de modes propres de forme de cavité nasale étant effectuées en appliquant une analyse en composantes principales.

7. Dispositif de commande comprenant au moins un processeur et au moins une mémoire comprenant un code de programme informatique, l'au moins une mémoire et le code de programme informatique étant configurés pour, avec l'au moins un processeur, amener le dispositif de commande à effectuer le procédé selon l'une quelconque des revendications précédentes 1 à 6.

8. Produit-programme informatique comprenant des instructions exécutables par ordinateur pour réaliser le procédé selon l'une quelconque des revendications précédentes 1 à 6 lorsque le programme est exécuté sur un ordinateur.

9. Support de stockage lisible par ordinateur comprenant des instructions exécutables par ordinateur pour réaliser le procédé selon l'une quelconque des revendications précédentes 1 à 6 lorsque le programme est exécuté sur un ordinateur.
